# EUROPEAN PATENT APPLICATION

(11) **EP 0 887 061 A1**
(43) Date of publication of application: **30.12.1998**
(21) Application number: 97110602.6
(22) Date of filing: 28.06.1997
(51) Int. Cl.: A61F 13/15, A61F 5/451

(54) **Faecal collector**

(71) Applicant: THE PROCTER & GAMBLE COMPANY, Cincinnati, Ohio 45202 (US)
(72) Inventor: Palumbo, Gianfranco, 61348 Bad Homburg (DE); D'Acchioli, Vincenzo, 65779 Kelkheim am Taunus (DE)
(74) Representative: Canonici, Jean-Jacques

(57) **Abstract**

The present invention relates to a faecal management device with an increased level of comfort. The faecal management device comprises a bag having an aperture, and an anatomically-shaped flange, which surrounds the aperture. The flange provides for adhesive attachment to the perianal area of the wearer. In particular, the bag comprises a wall material having an inner surface and an outer surface with the outer surface being provided with a non-woven layer. In another aspect of the present invention, the faecal management device is used in combination with a disposable diaper.

## Description

### Field of the invention

The present invention relates to a faecal management device having a softer tactile feel for increased wearer comfort and satisfaction.

### Background of the invention

Faecal management devices are known articles of manufacture that are designed to be worn principally by incontinence sufferers and infants. Such faecal management devices are attached to the anal region of the wearer and are intended to entrap and immediately contain faecal material and other bodily discharges. As a consequence, these devices are functionally effective in eliminating the problem of smearing on the skin of the wearer; in lessening epidermal irritation; in preventing contamination of articles such as clothing and bedding; and even in preventing the soiling of the carers themselves. Nevertheless, a problem often encountered during the use of such faecal management devices is that the constituent material of the outer surface of the devices tends to cause discomfort and in some cases, extreme discomfort to, for example, the bedridden wearer or to the infant.

Typically, the faecal management devices are made from a plastic material. For instance, US 3,577,989 details a disposable elimination-trapping bag for incontinence sufferers including a sack having an open-top portion, and a flange secured to the sack around the open-top portion. The elimination trapping bag is made from a light thin plastic. US 4,784,656 describes a receptacle for collecting faecal matter from incontinence sufferers. The faecal collector comprises a gasket, conduit means and a receptacle. The receptacle and conduit means are each formed from two sheets of odour barrier thermoplastic film that are heat sealed along their side edges, respectively. In a further embodiment, the receptacle and conduit means may also be made of any suitable fluid impervious, odour-barrier film or plastic. GB 2 152 387 describes a faecal collector for incontinence sufferers comprising a collection bag and a ring. The collection bag is preferably formed from a single sheet of odour-barrier thermoplastic film folded along a vertical midline to provide a pair of continuous panels. In another embodiment, the collection bag may be formed of any suitable thermoplastic film or film laminate. EP 0 245 064 A2 describes a faecal incontinence bag having flexible front and rear walls secured together around their periphery. The front wail has a hole for entry of the matter discharged by the wearer. Normally, the front and rear walls are made of synthetic plastic material such as PVC or multilayer films with high odour barrier properties. The surface of the front wall for contact with the wearer may be provided with a so-called "comfort layer", i.e., a layer of perforated or porous plastic material to prevent the bag from sticking to the wearer.

Plastic material, while functionally acceptable, is endowed with certain characteristics that are unsatisfactory and disagreeable to the wearer. The feel or texture of plastic material is particularly disturbing from the point of view of skin healthiness. Typically, the skin of incontinence sufferers and infants is especially sensitive and needs to be treated gently and with care. It has been recognised that the rubbing of plastic material from a faecal management device against the body of a wearer during wear can lead to occlusion, create hot clammy conditions and stickiness, reddening, skin rashes and perhaps, even lead to a more severe skin irritation and ultimately to the disgarding of the device. Therefore, a real consumer need can be identified for a faecal management device with superior cushioning qualities and a softer tactile feel.

The present invention addresses this need by providing the outer surface of the faecal management device with a non-woven layer. It has been found that the presence of this non-woven layer is uniquely advantageous and greatly enhances the softness of the faecal management device without adversely affecting its functionality. Furthermore, the faecal management device is aesthetically more pleasing, produces less crinkle during use and results in a high level of wearer and carer satisfaction in relation to skin healthiness.

In another aspect of the present invention, the faecal management device with its non-woven outer layer can be advantageously used with a disposable diaper. The prior art is actually silent on such a combination. JP 08-117,261 only teaches a diaper having a bag-like structure made of waterproof material at least at the anus facing portion, in which the bag-like structure has a slit or a hole having an adhesive coated on the periphery at the hip-contacting surface. The document, however, does not disclose two separate entities that work synergetically to isolate the skin of the wearer from the absorbent material of the diaper. In this manner, the constituent material of the outer surface of the faecal management device can be capitalised upon to reduce not only the problem of occlusion and epidermal irritations, but also contribute greatly to improved skin healthiness and lead to very satisfied wearers.

### Summary of the invention

A faecal management device constructed in accordance with the present invention comprises a bag having an aperture and an anatomically shaped flange surrounding the aperture for adhesive attachment to the perianal area of the wearer. The anatomically-shaped flange is attached to the bag. The bag comprises a wall material that has an outer surface and an inner surface. The outer surface is provided with a non-woven layer. The inner surface comprises a plastic film. The plastic film is preferably permeable to air and to vapour.

In a preferred embodiment of the present invention, the non-woven layer and the plastic film are in the form of a laminate. In another preferred embodiment of the present invention, the inner surface comprises a thermoplastic material. In yet another preferred embodiment, the inner surface comprises a second non-woven layer. In yet even a further embodiment of the present invention, the inner surface comprises the second non-woven layer and a plastic film. For all embodiments, the non-woven layer is preferably hydrophobic. For the case of the inner surface comprising a plastic film, the second non-woven layer can either be hydrophobic or hydrophilic and for the case of the inner surface not comprising a plastic film, the second non-woven layer can be hydrophobic. In addition, the non-woven layer can be treated with agents to improve its tactile feel and surface smoothness, and can be impregnated with a lotion to nourish and soften and protect the skin.

In another aspect of the present invention, the present faecal management device is used in combination with a disposable diaper.

### Brief description of the drawings

It is believed that the invention will be better understood from the foregoing description in conjunction with the accompanying drawings in which:
Figure 1 is a perspective view of a faecal management device according to the teachings of the present invention;
Figure 2 is a cross sectional view of the faecal management device as displayed in Figure 1;
Figure 3 shows a perspective view of the faecal management device in conjunction with a disposable diaper; and
Figure 4 is a partially cut-away perspective view of a disposable diaper embodying the present invention.

### Detailed description of the invention

As used herein, the term "layer" does not necessarily limit the inner surface and outer surface of the wall material of the faecal management device to a single stratum of material. The term "non-woven" can refer to continuous filaments, or fibre webs and batts that are strengthened by bonding using various techniques. Furthermore, the term "non-woven" can encompass loose and unadhered fibres that are applied to another web or film.

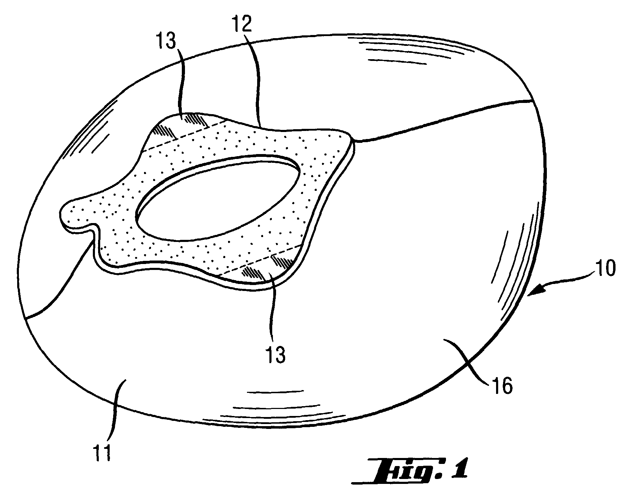

According to Figure 1, the faecal management device **10** of the present invention comprises a bag **11** having an aperture and an anatomically-shaped flange **12** surrounding the aperture for adhesive attachment to the perianal area of the wearer. The anatomically-shaped flange **12** is attached to the bag **11** according to means known to the man skilled in the art. Preferably, the anatomically-shaped flange **12,** which is substantially heart-shaped, is designed to closely follow the bodily contours of the wearer when in use. Furthermore, the flange **12** can be adapted to fit either a male or a female wearer. For a female wearer, the flange **12** at its front end portion comprises a projection that fits comfortably and snugly between the vulva and the anus, i.e., the perineum. For a male wearer, this projection is typically not present. The flange **12** comprises a body-compatible adhesive that is covered with a release means (not shown) in order to protect the adhesive layer. The adhesive can cover the entire surface of the flange **12** or can partially cover the entire surface. As is evident from Figure 1, the adhesive is not applied to the whole surface area of the flange **12**. Lobes **13** on either side of the flange **12** remain free of adhesive to facilitate more easily the removal of the release means. Before application of the faecal management device **10** to the perianal area of the wearer, the release means is removed.

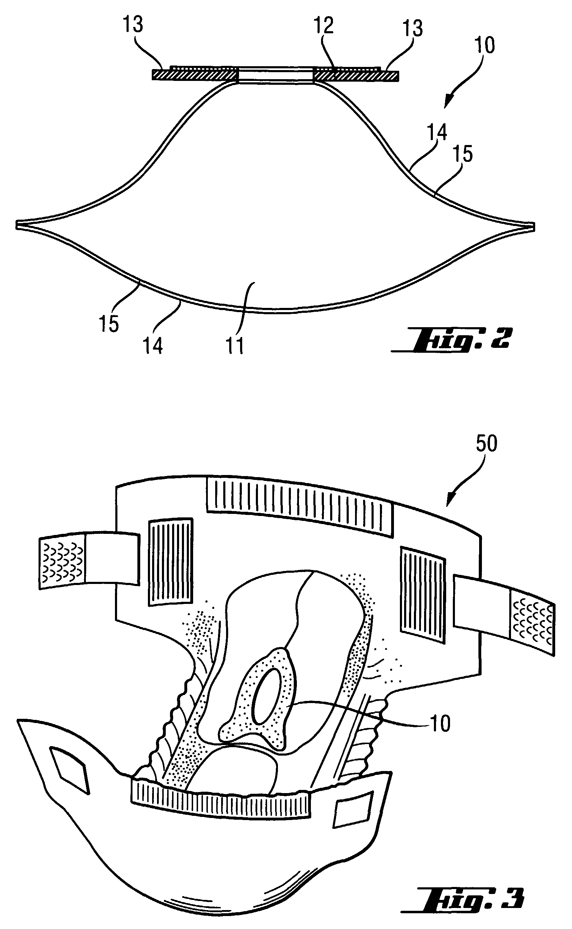

According to Figure 2, the bag **11** comprises a wall material, which has an outer surface **14** and an inner surface **15**; the outer surface **14** being provided with a non-woven layer. The non-woven layer can comprise felt fabrics, needlepunched fabrics, spunlaced fabrics, fluid jet entangled fabrics, air-laid fabrics, wet-laid fabrics, dry-laid fabrics, melt-blown fabrics, staple fibre carding fabrics, spunbonded fabrics, stitch-bonded fabrics, apertured fabrics, combinations of the above or the like. Such material layers present an uneven surface to the skin of the wearer and thus reduce significantly the problem of occlusion and greatly improve skin healthiness. The inner surface **15** of the bag **11** comprises a plastic film. The plastic film is preferably permeable to gases such as air and to vapour such as water vapour in order to avoid the problem of entrapment and condensation of moisture vapour given off by the body of the wearer and thus, the hot, clammy and uncomfortable conditions after a short period of use. In a preferred embodiment of the present invention, the non-woven layer and the plastic film are in the form of a laminate. The laminate can be formed by means known to the man skilled in the art.

In another preferred embodiment of the present invention, the inner surface **15** comprises a thermoplastic material. The thermoplastic material can be selected from among all types of hot-melt adhesives, polyolefins especially polyethylene, polypropylene, amorphous polyolefins, and the like; materials containing meltable components comprising fibres or polymeric binders including natural fibres such as cellulose - wood pulp, cotton, jute, hemp; synthetic fibres such as fibreglass, rayon, polyester, polyolefin, acrylic, polyamid, aramid, polytetrafluroethylene metal, polyimide; binders such as bicomponent high melt/low melt polymer, copolymer polyester, polyvinyl chloride, polyvinyl acetate/chloride copolymer, copolymer polyamide, materials comprising blends wherein some of the constituent materials are not meltable; air and vapour permeable materials including microporous films such as those supplied by EXXON Chemical Co., III, US under the designation EXXAIRE or those supplied by Mitsui Toatsu Co., Japan under the designation ESPOIR NO; and monolithic breathable materials such as Hytrel® available from DuPont and Pebax® available from ELF Atochem, France.

In yet another preferred embodiment of the present invention, the inner surface **15** comprises a second non-woven layer. The combination of the woven layer of the outer surface **14** and the second non-woven layer of the inner surface **15** results in a closed fibrous structure, which has a particularly pleasing sensation on contact with the skin of the wearer. In yet even a further embodiment of the present invention, the inner surface **15** comprises the second non-woven layer and a plastic film. The second non-woven layer of the inner surface **15** can lie either adjacent to the non-woven layer of the outer surface **14** with the plastic film lying furthest away from the non-woven layer or the second non-woven layer of the inner surface **15** can lie furthest away from the non-woven layer of the outer surface **14** with the plastic film being interposed therebetween.

For all embodiments of the present invention, the non-woven layer is preferably hydrophobic. As a consequence, fluid penetration is resisted through the outer surface **14** of the faecal management device **10**. For the case of the inner surface **15** comprising a plastic film, the second non-woven layer can either be hydrophobic or hydrophilic and for the case of the inner surface **15** not comprising a plastic film, the second non-woven layer can be hydrophobic. Typically, the non-woven layer is treated with a surface active material, such as a fluorochemical or other hydrophobic finishings, to provide the requisite hydrophobicity. The non-woven layer, however, may equally be treated with coatings of liquid impervious materials such as hot-melt adhesives or coatings of silicone or other hydrophobic compounds such as rubbers and vegetable and mineral waxes or it may be physically treated using nano-particulates or plasma coating techniques, for example.

The non-woven layer can also be treated with agents to improve the tactile perceivable softness of the outer surface **14** of the wall material. The agents include but are not limited to vegetable, animal or synthetic oils, silicone oils and the like. The presence of these agents are known to impart a silky or flannel-like feel to the non-woven layer without rendering it greasy or oily to the tactile sense of the wearer. Additionally, surfactant material, including anionic, non-anionic, cationic and non-cationic surfactants, may be added to further enhance softness and surface smoothness.

Furthermore, the non-woven layer may be impregnated with a lotion to provide desirable therapeutic or protective coating lotion benefits. The lotion coating on the outer surface **14** of the wall material is transferable to the skin of the wearer by normal contact and wearer motion and/or body heat. Generally, mineral oil in the form of a lotion is recognised as being effective in imparting a soothing, protective coating to the skin of the wearer. It is also possible to impregnate the non-woven layer with a solid oil phase of cream formulation or to incorporate into the non-woven layer an array of pressure- or thermal- or hydro- rupturable capsules containing for example, baby oil.

The faecal management device **10** of the present invention has been found to be particularly useful and beneficial when used in conjunction with a disposable diaper **50** - refer to Figure 3. The faecal management device **10** is first positioned in the perianal area of the wearer before the disposable diaper **50** is applied. In particular, the diaper **50** is positioned over the faecal management device **10** and fastened in a conventional manner around the body of the wearer. It has been found that, in addition, to providing excellent separation between urine and faecal material, the combined faecal management device **10** and diaper **50** system actually reduces skin irritation. In effect, the presence of the faecal management device **10** permits the formation of a separation layer between the skin of the wearer and the diaper **50**, i.e., a part of the absorbent core **58** of the diaper **10**. The diaper **50** can be of the conventional type (an embodiment of which is described below although not a limiting example by any means) or can be adapted to contain in an effective and comfortable manner the faecal management device **10** according to the teachings of the present invention.

As used herein, the term "disposable diapers" refers to articles which absorb and contain body exudates; and more specifically, refers to articles which are placed against or in proximity to the body of the wearer to absorb and contain the various exudates discharged from the body and which are intended to be discarded after a single use (i.e., they are not intended to be laundered or otherwise restored or reused) and, preferably, to be recycled, composted or otherwise disposed of in an environmentally compatible manner. As used herein, the term "diaper" refers to a garment generally worn by infants or incontinence sufferers that is drawn up between the legs and fastened about the waist of the wearer.

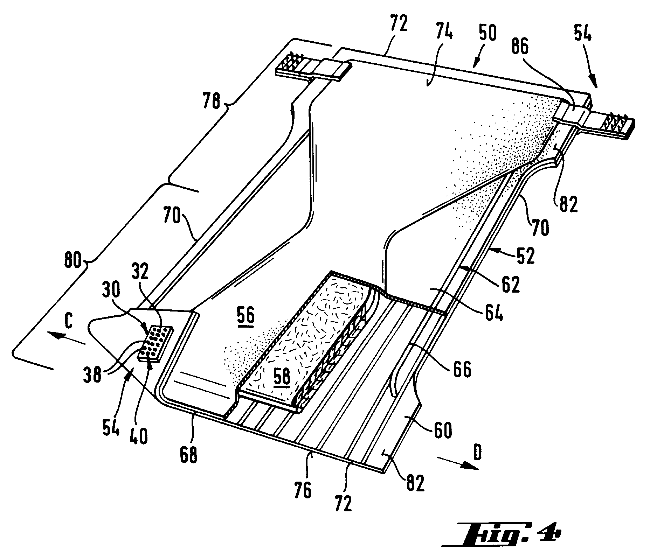

Figure 4 is a partially cut-away perspective view of a diaper **50** embodying the present invention prior to it being placed on the wearer over the faecal management device **10**. As is visible from Figure 4, a preferred diaper **50** comprises a body portion **52** and a refastenable mechanical fastening device **54**. A preferred body portion **52** comprises a liquid pervious topsheet **56**, an absorbent core **58**, a liquid impervious backsheet **60**, and elastically contractible leg cuffs **62**; each leg cuff **62** preferably comprising a side flap **64** and one or more elastic members **66**. For simplicity purposes, only one elastic member **66** is shown in the side flap **64**. While the topsheet **56**, the absorbent core **58**, the backsheet **60**, the side flaps **64**, and the elastic members **66** may be assembled in a variety of well-known configurations, a preferred disposable diaper configuration is shown and generally described in US 3,860,003. In this preferred diaper configuration, the backsheet **60** is joined to the topsheet **56**; the absorbent core **58** is positioned between the topsheet **56** and the backsheet **60**; the side flaps **64** extend outwardly from and along each side edge of the absorbent core **58**; and the elastic member **66** is operatively associated with each side flap **64**.

Figure 4 shows the body portion **52** in which the topsheet **56** and the backsheet **60** are coextensive and have length and width dimensions generally larger than those of the absorbent core **58**. The topsheet **56** is superposed on the backsheet **60** thereby forming the periphery **68** of the body portion **52**. The periphery **68** defines the outer perimeter or in other words the outer extent of the body portion **52**. The periphery comprises the longitudinal side edges **70** and lateral end edges **72**. In the longitudinal direction, the diaper **50** has a first end region **78** and a second end region **80**.

The body portion **52** has an inside surface **74** and an outside surface **76**. In general, the outside surface **76** of the diaper **50** extends from one edge **72** to the other end edge **72** of the diaper **50** and from one longitudinal side edge **70** to the other longitudinal side edge **70** of the diaper **50**. When a backsheet **60** is used, it typically forms the outside surface **76** of the body portion **52**. The inside surface **74** is that surface of the diaper **50** opposite the outside surface **76** and in the embodiment shown is typically formed by the topsheet **56**. In general, the inside surface **74** of the diaper **50** is that surface coextensive with the outside surface **76** and which is for the greater part in contact with the wearer when the diaper **50** is worn.

The diaper **50** has a first end region **78** and a second end region **80** extending from the lateral end edges **72** of the diaper periphery **68** towards the lateral centreline of the diaper **50**. Both the first end region **78** and the second end region **80** extend a distance of about one-half the length of the diaper **50** such that the end regions **78**, **80** comprise each half of the diaper **50**. Both the first end region **78** and the second end region **80** have panels **82**. The panels **82** are those portions of the first end region **78** and the second end region **80** which overlap when the diaper **50** is fastened about the waist of the wearer. The extent to which the end regions **78** overlap and thus the extent to which the panels **82** are formed, will depend on the overall dimensions and shape of the diaper **50** and the size of the wearer.

The absorbent core **58** of the body portion **52** may be any absorbent means which is generally compressible, conformable, non-irritating to the skin of the wearer, and capable of absorbing and retaining liquids such as urine and other certain bodily discharges. The absorbent core **58** may be manufactured in a variety of sizes and shapes (for example, rectangular, hour-glass, "T"-shaped, asymmetric, etc.) and from a wide variety of liquid absorbent materials commonly used in disposable diapers and other absorbent articles such as comminuted wood pulp which is generally referred to as airfelt. Examples of other suitable absorbent materials include creped cellulose wadding, meltblown polymers including coform, crosslinked cellulosic fibers, tissue including tissue wraps, absorbent foams, absorbent sponges, superabsorbent polymers, absorbent gelling materials, or any equivalent materials or combinations of materials. The configuration and construction of the absorbent core **58** may also be varied (for example, the absorbent core **58** may have varying caliper zones, hydrophilic gradients, superabsorbent gradients, or lower average density and lower average basis weight acquisition zones; or may comprise one or more layers or structures). The total absorbent capacity of the absorbent core **58** should, however, be compatible with the design loading and the intended use of the pull-on diaper. Further, the size and absorbent capacity of the absorbent core **58** may be varied to accommodate wearers ranging from infants to adults.

The backsheet **60** is impervious to liquids (for example, urine) and is preferably manufactured from a thin plastic film, preferably a thermoplastic film, although other flexible liquid impervious materials may also be used. As used herein, the term "flexible" refers to materials which are compliant and which will readily conform to the general shape and contours of the human body. The backsheet **60** prevents the exudates absorbed and contained in the absorbent core **58** from soiling articles which are in contact with the diaper **50** such as undergarments and bedding. The backsheet **60** may thus comprise polymeric films such as thermoplastic films of polyethylene or polypropylene, or composite materials such as film-coated non-woven material. For economic, aesthetic and ecological reasons, the backsheet **60** has an average nominal caper, i.e., calculated caper, of less than about 0.051 millimetres, more preferably a calculated caliper of from 0.020 millimetres to 0.036 millimetres. Preferably, the backsheet **60** is a flexible polyethylene film. As used herein, the term "polyethylene" film refers to films which are essentially made of polyethylene, however, it is understood that polyethylene film will contain a variety of additives to provide characteristics like opacity, strength requirements, colour, or any other desired characteristic that can be achieved through adding minor amounts of other substances than polyethylene into the films. The total amount of additives should be less than 45 percent, preferably less than 15 percent, by weight of film materials. Particularly, for opacity of the film, titanium dioxide is commonly used in a range of 2 to 6 percent, preferably 3.5 to 4.8 percent, by weight of the film. Exemplary films are manufactured by Tredegar Industries, Inc. of Terre Haute, Ind., USA or BP-Chemical PlasTec Rotbuchenstrasse 1, D-8000 München, Germany.

The backsheet **60** is preferably textured to provide a more clothlike appearance. Further, the backsheet **60** may also permit vapours to escape from the absorbent core **58** while still preventing exudates from passing through the backsheet **60** by, for example, being supplied with microapertures. The size of the backsheet **60** is dictated by the size of the absorbent core **58** and the exact diaper design selected.

The topsheet **56** of the body portion **52** of the present invention is compliant, soft feeling and non-irritating to the skin of the wearer. Further, the topsheet **56** is liquid pervious permitting liquids (for example, urine) to readily penetrate through its thickness. A suitable topsheet **56** may be manufactured from a wide range of materials, such as porous foams, reticulated foams, apertured films; or woven or non-woven webs of natural fibres (for example, wood or cotton fibres) or from a combination of natural and synthetic fibres. Preferably, it is made of a material that isolates the skin of the wearer from liquids retained in the absorbent core **58**.

There are a number of manufacturing techniques which may be used to manufacture the topsheet **56**. For example, the topsheet **56** may be a non-woven web of fibres. When the topsheet **56** comprises a non-woven web, the web may be spunbonded, carded, wet-laid, melt-blown, hydroentangled, hydroformed, combinations of the above, or the like. An exemplary topsheet **56** is carded and thermally bonded by means well-known to those skilled in the fabric art and comprise staple length polypropylene fibres having a denier of about 2.2 and has a basis weight from about 15 to about 30 grammes per square metre. As used herein, the term "staple length fibres" refer to those fibres having a length of at least 16 millimetres. A suitable topsheet **56** is manufactured by, for example, Veratec Inc., a division of International Paper Company, of Walpole, Mass., USA. A topsheet **56** particularly preferred for incontinence garments comprises a formed thermoplastic film.

### GLOSSARY

- 10: Faecal management device
- 11: Bag
- 12: Flange
- 13: Lobes
- 14: Outer surface
- 15: Inner surface
- 50: Diaper
- 52: Body portion
- 54: Mechanical fastening device
- 56: Topsheet
- 58: Absorbent core
- 60: Backsheet
- 62: Leg cuffs
- 64: Side flaps
- 66: Elastic members
- 68: Periphery of body portion
- 70: Longitudinal side edges
- 72: Lateral end edges
- 74: Inside surface of body portion
- 76: Outside surface of body portion
- 78: First end region
- 80: Second end region
- 82: Panels

## Claims

1. Faecal management device (10) comprising a bag (11), said bag (11) having an aperture and an anatomically-shaped flange (12) surrounding said aperture for adhesive attachment to perianal area of wearer, said anatomically-shaped flange (12) being attached to said bag (11), said bag (11) comprising a wall material,
characterised in that
said wall material has an outer surface (14) and an inner surface (15) with said outer surface (14) being provided with a non-woven layer.

2. Faecal management device (10) according to claim 1 wherein said inner surface (15) comprises a plastic film.

3. Faecal management device (10) according to claim 2 wherein said plastic film is permeable to air and to vapour.

4. Faecal management device (10) according to any of the preceding claims wherein said non-woven layer and said plastic film are in the form of a laminate.

5. Faecal management device (10) according to claims 1 and 2 wherein said inner surface (15) comprises a thermoplastic material.

6. Faecal management device (10) according to claim 1 wherein said inner surface (15) comprises a second non-woven layer.

7. Faecal management device (10) according to claim 2 wherein said inner surface (15) further comprises a second non-woven layer.

8. Faecal management device (10) according to any of the preceding claims wherein said non-woven layer is hydrophobic.

9. Faecal management device (10) according to claims 6 or 7 wherein said second non-woven layer is hydrophobic.

10. Faecal management device (10) according to claim 7 wherein said second non-woven layer is hydrophilic.

11. Faecal management device (10) according to any of the preceding claims wherein said non-woven layer is softened with agents to enhance softness.

12. Faecal management device (10) according to any of the preceding claims wherein said non-woven layer is impregnated with a lotion.

13. Use of a faecal management device (10) according to any of the preceding claims in combination with a disposable diaper (50).

14. Use of a faecal management device (10) according to claim 13 whereby said faecal management device (10) is first positioned in the perianal area of the wearer and then said disposable diaper (50) is positioned over said faecal management device (10) and fastened in a conventional manner around body of said wearer.

15. Use of a faecal management device (10) according to claims 13 and 14 wherein said faecal management device (10) provides a separation layer between skin of said wearer and said disposable diaper (50).
